# EUROPEAN PATENT SPECIFICATION

(11) **EP 2 582 361 B1**
(45) Date of publication and mention of the grant of the patent: **10.09.2014**
(21) Application number: 11728238.4
(22) Date of filing: 21.06.2011
(51) Int. Cl.: A61K 9/20, A61K 31/4184

(54) **MULTILAYER PHARMACEUTICAL TABLET COMPRISING TELMISARTAN AND A DIURETIC**
MEHRSCHICHTIGE PHARMAZEUTISCHE TABLETTE MIT TELMISARTAN UND EINEM DIURETIKUM
COMPRIMÉ PHARMACEUTIQUE MULTICOUCHE COMPRENANT DU TELMISARTAN ET UN DIURÉTIQUE

(30) Priority: 21.06.2010 WO PCT/EP2010/058754
(43) Date of publication of application: 24.04.2013
(73) Proprietor: Krka Tovarna Zdravil, D.D., Novo Mesto, 8501 Novo Mesto (SI)
(72) Inventor: SEDMAK, Gregor, 1000 Ljubljana (SI); VRECER, Franc, 8351 Straza pri Novem mestu (SI)
(74) Representative: UEXKÜLL & STOLBERG
(86) International application number: PCT/EP2011/060381
(87) International publication number: WO 2011/161123

(56) References cited:
- WO-A2-2007/060170
- WO-A2-2009/058950

## Description

The invention relates to a multilayer pharmaceutical tablet comprising telmisartan and a diuretic. It further relates to a process for the preparation of a multilayer pharmaceutical tablet.

### Background of the invention

Telmisartan, the chemical name of which is 2-(4-{[4-methyl-6-(1-methylbenzimidazol-2-yl)-2-propylbenzimidazol-1-yl]-methyl}-phenyl)-benzoic acid or 4'-[(2-n-propyl-4-methyl-6-(1-methylbenzimidazol-2-yl)benzimidazol-1-yl)methyl]biphenyl-2-carboxylic acid, is a non-peptide angiotensin II receptor type AT₁ antagonist useful for the treatment of hypertension that was originally disclosed in EP 0 502 314 A1 and J. Med. Chem. 36 (25), 4040-4051 (1993). Telmisartan is commercially available in particular in its free acid form, which is poorly soluble in neutral or acidic media. Thus, telmisartan is typically formulated together with a basic agent or in the form of a basic salt for improved solubility.

Diuretics such as amiloride, chlorothalidone, furosemide, hydrochlorothiazide, indapamide or piretanide are therapeutic agents which can also be used in the treatment of hypertension. It is also known to use combinations of angiotensin II receptor antagonists like telmisartan and diuretics like hydrochlorothiazide in the treatment of hypertension in order to achieve a synergistic effect. It is generally desirable for such combination formulations that the diuretic is dissolved more rapidly than the angiotensin II receptor antagonist.

Hydrochlorothiazide (HCTZ), the chemical name of which is 6-chloro-1,1-dioxo-3,4-dihydro-2H-1,2,4-benzothiadiazine-7-sulfon-amide, is a diuretic which is stable in neutral and relatively weak acidic environments, but poorly stable in alkaline environments. In particular, HCTZ is incompatible with basic agents that are commonly used together with the free acid form of telmisartan. This incompatibility is problematic where combination formulations of telmisartan and a base-labile diuretic such as HCTZ are concerned.

Different approaches have been pursued in order to address the incompatibility problem. A classical approach is to coat diuretic particles with water soluble polymers like hydroxypropylcellulose, hydroxypropylmethylcellulose or polyvinylpyrrolidone. However, this measure has been found not to sufficiently protect the diuretic from degradation when formulated together with telmisartan as a compressed tablet. Moreover, the polymeric coating typically results in a reduced dissolution rate of the diuretic.

WO 2003/059327 A1 discloses a bilayer tablet comprising a first tablet layer containing telmisartan in a dissolving tablet matrix and a second tablet layer containing a diuretic in a disintegrating tablet matrix. WO 2004/028505 A1, WO 2004/ 096215 A1, WO 2006/063737 A1 and WO 2007/060170 A2 describe further embodiments of bilayer tablets. The telmisartan containing layer is based on a composition that is generally prepared using spray-drying or fluid bed granulation. The diuretic containing layer is based on a composition that is particularly prepared by mixing or granulation of a diuretic with the constituents of a disintegrating tablet matrix. Among these constituents are a number of specialty excipients such as dry binders, wet granulation binders, and disintegrants.

WO 2007/144175 A2 discloses a pharmaceutical composition comprising first units in the form of granules, pellets or tablet cores comprising telmisartan and second units in the form of granules, pellets, or tablet cores comprising HCTZ. The first and second units can be compressed into tablets together with a suitable carrier such that the units are substantially evenly distributed throughout the tablets. The first and second units are optionally coated. In particular, a separating coating of the first units is contemplated. Furthermore, polyvinylpyrrolidone should be incorporated into the HCTZ containing part of the composition in order to achieve better stability.

WO2009/058950 discloses formulations comprising telmisartan and hydrochlorothiazide in a bilayer form wherein the first layer comprises telmisartan in a disintegrating matrix comprising a basic agent and the second layer comprises hydrochlorothiazide in a dissolving or disintegrating matrix. WO2009/058950 also discloses embodiments where the second tablet layer does not contain a disintegrant and comprises granules with the active agent (hydrochlorothiazide), filler (mannitol) and a binder (povidone) and an extragranular phase (mannitol and magnesium stearate).

In the pharmaceutical compositions of the prior art, complex formulations of the angiotensin II receptor antagonist and the diuretic are necessary to address the incompatibility of the active agents and stabilize the compositions. These complex formulations require the use of substantial amounts of expensive specialty excipients. Moreover, preparation of these formulations involves complex and costly processing steps such as wet granulation.

It is therefore an object of the present invention to provide a multilayer pharmaceutical tablet comprising telmisartan and a diuretic in a fixed dosage form avoiding these drawbacks and which not only exhibits high chemical and physical stability as well as an appropriate dissolution profile and bioavailability, but which also can be prepared from inexpensive excipients and can be manufactured using a simple and economical process.

This object is surprisingly achieved by the multilayer pharmaceutical tablet described herein below. The invention also relates to a process for the preparation of a multilayer pharmaceutical tablet comprising telmisartan and a diuretic.

### Summary of the invention

In one aspect, the invention relates to a multilayer pharmaceutical tablet comprising
(a) at least one first tablet layer comprising
   1 to 50 wt.-% telmisartan or a pharmaceutically acceptable salt thereof
   by weight of the first tablet layer; and
(b) at least one second tablet layer comprising
   1 to 50 wt.-% of a diuretic and
   50 to 99 wt.-% of at least one filler
   by weight of the second tablet layer, wherein the combined weight of the diuretic and the at least one filler is at least 87 wt.-% by weight of the second tablet layer.
wherein the second tablet layer comprises
granules comprising the diuretic, at least one filler, at least one binder and optionally other pharmaceutically acceptable excipients; and
an extragranular phase comprising at least one filler, at least one lubricant and optionally other pharmaceutically excipients;
wherein the weight ratio of the at least one filler of the granules to the at least one filler of the extragranular phase is ranging from 1:1 to 1:10.

Furthermore, the invention also relates to a process for the preparation of a multilayer pharmaceutical tablet comprising at least one layer comprising telmisartan and at least one layer comprising a diuretic, the process comprising
(a) providing a first tablet layer composition comprising telmisartan;
(b) providing a second tablet layer composition comprising a diuretic by
   (i) granulating the diuretic with at least one filler, at least one binder and optionally other pharmaceutically acceptable excipients to obtain granules; and
   (ii) blending the obtained granules with at least one filler and optionally other pharmaceutically acceptable excipients; wherein the weight ratio of the at least one filler of the granules to the at least one filler used in step (b)(ii) is ranging from 1:1 to 1:10;
(c) optionally providing one or more further tablet layer compositions; and
(d) compressing said tablet layer compositions to result in the multilayer pharmaceutical tablet.

Moreover, the invention also relates to a process for the preparation of a multilayer pharmaceutical tablet comprising at least one layer comprising telmisartan and at least one layer comprising a diuretic, the process comprising
(a) providing a first tablet layer composition comprising telmisartan;
(b) providing a second tablet layer composition comprising a diuretic by
   (i) dry granulating the diuretic with at least one filler and optionally other pharmaceutically acceptable excipients to obtain a granulate; and
   (ii) blending the obtained granulate with at least one filler and optionally other pharmaceutically acceptable excipients;
   wherein the weight ratio of the at least one filler of the granulate to the at least one filler used in step (b)(ii) is ranging from 1:1 to 1:10;
(c) optionally providing one or more further tablet layer compositions; and
(d) compressing said tablet layer compositions to result in the multilayer pharmaceutical tablet.

### Detailed description of the invention

In a first aspect, the invention relates to a multilayer pharmaceutical tablet comprising
(a) at least one first tablet layer comprising
   1 to 50 wt.-% telmisartan or a pharmaceutically acceptable salt thereof
   by weight of the first tablet layer; and
(b) at least one second tablet layer comprising
   1 to 50 wt.-% of a diuretic and
   50 to 99 wt.-% of at least one filler
by weight of the second tablet layer, wherein the combined weight of the diuretic and the at least one filler is at least 87 wt.-% by weight of the second tablet layer.
wherein the second tablet layer comprises
granules comprising the diuretic, at least one filler, at least one binder and optionally other pharmaceutically acceptable excipients; and
an extragranular phase comprising at least one filler, at least one lubricant and optionally other pharmaceutically excipients;
wherein the weight ratio of the at least one filler of the granules to the at least one filler of the extragranular phase is ranging from 1:1 to 1:10.

As used herein, the term "multilayer pharmaceutical tablet" refers to a pharmaceutical tablet which is made up of at least two distinct layers, such as at least two layers, at least three layers, at least four layers, at least five layers, etc., with the individual layers being arranged one on top of another. The multilayer tablet generally has a sandwich-like appearance because the edges of each layer are exposed. Typically, adjacent layers of the tablet will be of different composition. It has to be understood that the terms "first tablet layer" and "second tablet layer" as used herein refer to tablet layers having a particular composition. However, these terms do not necessarily reflect the order in which the layers are arranged in the tablet.

It has surprisingly been found that the multilayer pharmaceutical tablet of the invention exhibits a dissolution profile that is comparable to the one typically found for tablets containing telmisartan in a dissolving tablet matrix and a diuretic in a disintegrating tablet matrix.

The first tablet layer comprises telmisartan or a pharmaceutically acceptable salt thereof. Telmisartan is typically employed in its free acid form although pharmaceutically acceptable salts can also be used. The first tablet layer preferably comprises 3 to 50 wt.-%, particularly 5 to 35 wt.-%, more preferably 10 to 20 wt.-% of telmisartan or a pharmaceutically acceptable salt thereof.

According to one embodiment, the first tablet layer comprises telmisartan and a basic agent. The term "basic agent" as used herein refers to a substance which is characterized in that a 3 wt.=% aqueous solution thereof has a pH value of at least 8.0. Suitable basic agents include ammonia, NaOH, KOH, Ca(OH)₂, Na₂CO₃, K₂CO₃, NaHCO₃, KHCO₃, Na₃PO₄, K₃PO₄, Na₂HPO₄, K₂HPO₄, choline, *tert*-butylamine, ethanolamine, meglumine, piperazine, diethylamine, L-arginine and mixtures thereof. Alkali metal hydroxides such as NaOH and KOH, amino sugars such as meglumine and mixtures thereof are preferred basic agents. It is particularly preferred that the basic agent comprises a mixture of an alkali metal hydroxide such as NaOH or KOH and an amino sugar such as meglumine in a weight ratio of 1:1 to 1:10, more particularly 1:2 to 1:5, more preferably 1:3 to 1:4, most preferably about 1:3.5. The first tablet layer preferably comprises 0.25 to 20 wt.-%, particularly 1 to 15 wt.-%, more preferably 2 to 10 wt.-% of basic agent.

The telmisartan can be amorphous or crystalline. As used herein, the term "amorphous" includes amorphous and partly crystallized forms. Amorphous telmisartan can be obtained by methods generally known in the art such as freeze drying of aqueous solutions, coating of carrier particles by a solution of telmisartan in a fluidized bed, and solvent deposition on sugar pellets or other carriers.

According to another embodiment, the first tablet layer comprises a pharmaceutically acceptable salt of telmisartan. Suitable pharmaceutically acceptable salts include alkaline salts such as the sodium salt of telmisartan. The pharmaceutically acceptable salt of telmisartan can be amorphous or crystalline.

In addition to telmisartan or a pharmaceutically acceptable salt thereof, the first tablet layer can comprise pharmaceutically acceptable excipients. The term "pharmaceutical acceptable excipient" as used herein refers to additives useful for converting pharmacologically active compounds into pharmaceutical dosage forms which are suitable for administration to patients. Excipients suitable for use in the first tablet layer include fillers, binders, surfactants, crystallization retarders, lubricants, glidants and coloring agents. Other pharmaceutically acceptable excipients can also be included.

The first tablet layer typically comprises at least one filler. Water-soluble fillers are generally preferred. Suitable fillers for use in the first tablet layer include monosaccharides, oligosaccharides and sugar alcohols such as glucose, fructose, saccharose, lactose (anhydrous and monohydrate), raffinose, trehalose, dextrates, mannitol, erythritol, sorbitol, maltitol, xylitol and lactitol, compressible sugar, calcium hydrogen phosphate, calcium carbonate, calcium lactate and mixtures thereof. Preferred fillers are monosaccharides and oligosaccharides such as glucose, fructose, saccharose and lactose, sugar alcohols such as mannitol, erythritol, sorbitol, maltitol, xylitol and lactitol and mixtures thereof. Lactose, sorbitol and mixtures thereof are particularly preferred. It is particularly preferred that the filler comprises a monosaccharide or oligosaccharide such as lactose and a sugar alcohol such as sorbitol in a weight ratio of 1:1 to 1:10, particularly 1:2 to 1:5, most preferably about 1:2.5.

The first tablet layer preferably comprises 30 to 95 wt.-%, particularly 60 to 90 wt.-%, more preferably 70 to 80 wt.-% of filler.

Suitable binders for use in the first tablet layer include povidone (polyvinylpyrrolidone), copovidone (vinylpyrrolidone-vinylacetate copolymer), cellulose powder, crystalline cellulose, microcrystalline cellulose, siliconized microcrystalline cellulose, cellulose derivatives such as hydroxymethylcellulose, hydroxyethylcellulose, hydroxypropylcellulose and hydroxypropylmethylcellulose, starch, pregelatinized starch, polymethacrylates and mixtures thereof. Povidone is particularly preferred. The first tablet layer preferably comprises 1 to 30 wt.-%, particularly 2 to 10 wt.-%, more preferably 3 to 7 wt.-% of binder.

Suitable surfactants for use in the first tablet layer include anionic, cationic, ampholytic and non-ionic surfactants such as sodium lauryl sulfate, cetrimide, N-dodecyl-N,N-dimethyl-betaine, polysorbates (such as Tweens®), poloxamers and mixtures thereof. Non-ionic surfactants such as polysorbates and poloxamers are preferred. The first tablet layer preferably comprises 0 to 30 wt.-% like 1 to 30 wt.-%, particularly 2 to 10 wt.-%, more preferably 3 to 7 wt.-% of surfactant.

Suitable crystallization retarders for use in the first tablet layer include povidone, copovidone, crospovidone, carboxymethylcellulose sodium, hydroxypropylcellulose and hydroxypropylmethylcellulose. The first tablet layer preferably comprises 0 to 10 wt.-% like 0.1 to 10 wt.-%, particularly 0.25 to 5 wt.-%, more preferably 0.5 to 2 wt.-% of crystallization retarder.

Suitable lubricants for use in the first tablet layer include stearic acid and stearic acid salts such as magnesium stearate, magnesium palmitate and magnesium oleate, hydrogenated vegetable oil, hydrogenated castor oil, talc, sodium stearyl fumarate, macrogols and mixtures thereof. Stearic acid, magnesium stearate and hydrogenated vegetable oil are particularly preferred. The first tablet layer preferably comprises 0.1 to 10 wt.-%, particularly 0.25 to 5 wt.-%, more preferably 0.5 to 2 wt.-% of lubricant.

Suitable glidants for use in the first tablet layer include colloidal silicon dioxide and magnesium trisilicate. The first tablet layer preferably comprises 0 to 10 wt.-% like 0.1 to 10 wt.-%, particularly 0.25 to 5 wt.-%, more preferably 0.5 to 2 wt.-% of glidant.

Suitable coloring agents for use in the first tablet layer include dyes and pigments such as iron oxide and titanium oxide. The first tablet layer preferably comprises 0 to 1 wt.-% like 0.001 to 1 wt.-%, particularly 0.01 to 0.5 wt.-%, more preferably 0.05 to 0.2 wt.-% of coloring agent.

According to a particular embodiment, the first tablet layer comprises
(aa) 3 to 50 wt.-%, particularly 5 to 35 wt.-%, more preferably 10 to 20 wt.-% of telmisartan or a pharmaceutically acceptable salt thereof;
(bb) 0.25 to 20 wt.-%, particularly 1 to 15 wt.-%, more preferably 2 to 10 wt.-% of basic agent;
(cc) 30 to 95 wt.-%, particularly 60 to 90 wt.-%, more preferably 70 to 80 wt.-% of filler;
(dd) 1 to 30 wt.-%, particularly 2 to 10 wt.-%, more preferably 3 to 7 wt.-% of binder;
(ee) 0 to 30 wt.-%, particularly 2 to 10 wt.-%, more preferably 3 to 7 wt.-% of surfactant;
(ff) 0 to 10 wt.-%, particularly 0.25 to 5 wt.-%, more preferably 0.5 to 2 wt.-% of lubricant; and
(gg) 0 to 1 wt.-%, particularly 0.01 to 0.5 wt.-%, more preferably 0.05 to 0.2 wt.-% of coloring agent.

According to another preferred embodiment, the first tablet layer comprises
(aa) 3 to 50 wt.-%, particularly 5 to 35 wt.-%, more preferably 10 to 20 wt.-% of telmisartan or a pharmaceutically acceptable salt thereof;
(bb) 0.25 to 20 wt.-%, particularly 1 to 15 wt.-%, more preferably 2 to 10 wt.-% of basic agent, wherein said basic agent is preferably selected from NaOH, KOH, Ca(OH)₂, Na₂CO₃, K₂CO₃, NaHCO₃, KHCO₃, Na₃PO₄, K₃PO₉, Na₂HPO₄, K₂HPO₄, choline, *tert*-butylamine, ethanolamine, meglumine, piperazine, diethylamine, L-arginine and mixtures thereof, and is more preferably selected from NaOH, meglumine and mixtures thereof;
(cc) 30 to 95 wt.-%, particularly 60 to 90 wt.-%, more preferably 70 to 80 wt.-% of filler, wherein said filler is preferably selected from monosaccharides and oligosaccharides such as glucose, fructose, saccharose and lactose, sugar alcohols such as mannitol, erythritol, sorbitol, maltitol, xylitol and lactitol and mixtures thereof, and is more preferably selected from lactose, sorbitol and mixtures thereof;
(dd) 1 to 30 wt.-%, particularly 2 to 10 wt.-%, more preferably 3 to 7 wt.-% of binder, wherein said binder is preferably selected from povidone, copovidone cellulose powder, crystalline cellulose, microcrystalline cellulose, siliconized microcrystalline cellulose, cellulose derivatives such as hydroxymethylcellulose, hydroxyethylcellulose, hydroxypropylcellulose and hydroxypropylmethylcellulose, starch, pregelatinized starch, polymethacrylates and mixtures thereof, and is more preferably selected from povidone;
(ee) 0 to 10 wt.-%, particularly 0.25 to 5 wt.-%, more preferably 0.5 to 2.5 wt.-% of lubricant, wherein said lubricant is preferably selected from stearic acid and stearic acid salts such as magnesium stearate, magnesium palmitate and magnesium oleate, hydrogenated vegetable oil, hydrogenated castor oil, talc, sodium stearyl fumarate, macrogols and mixtures thereof, and is more preferably selected from magnesium stearate, sodium stearyl fumarate and mixtures thereof; and
(ff) 0 to 1 wt.-%, particularly 0.01 to 0.5 wt.-%, more preferably 0.05 to 0.2 wt.-% of coloring agent.

The second tablet layer comprises a diuretic and at least one filler. Preferred diuretics include amiloride, chlorothalidone, furosemide, hydrochlorothiazide, indapamide or piretanide. Hydrochlorothiazide (HCTZ) is particularly preferred.

The diuretic has preferably an average particle size of less than 80 µm, particularly less than 50 µm, more preferably less than 30 µm. The term "average particle size" as used herein refers to the volume mean diameter of particles. The volume mean diameter can be determined by laser light scattering for instance using a Malvern Mastersizer Apparatus MS 2000 equipped with a Hydro S dispersion unit.

The second tablet layer comprises at least one filler, wherein the combined weight of the diuretic and the at least one filler is at least 87 wt.-% by weight of the second tablet layer. It is particularly preferred that the combined weight of the diuretic and the filler is at least 90 wt.-%, more preferably at least 93 wt.-%, further more preferably at least 95 wt.-%, like at least 96 wt.-% or at least 97 wt.-% such as at least 98 wt.-%, and most preferably at least 99 wt.-% by weight of the second tablet layer.

Water-soluble fillers are generally preferred. Suitable fillers for use in the second tablet layer include monosaccharides, oligosaccharides and sugar alcohols such as glucose, fructose, saccharose, lactose (anhydrous and monohydrate), raffinose, trehalose, dextrates, mannitol, erythritol, sorbitol, maltitol, xylitol and lactitol, compressible sugar, calcium hydrogen phosphate, calcium carbonate, sodium carbonate in anhydrous or hydrated form, sodium hydrogen carbonate, calcium lactate and mixtures thereof. Preferred fillers are monosaccharides and oligosaccharides such as glucose, fructose, saccharose and lactose, sugar alcohols such as mannitol, erythritol, sorbitol, maltitol, xylitol and lactitol and mixtures thereof. In particular, sugar alcohols like mannitol, erythritol, sorbitol, maltitol, xylitol and lactitol and mixtures thereof are used as a filler in the second tablet layer. Mannitol is a particularly preferred filler of the second tablet layer. The second tablet layer preferably comprises 50 to 99 wt.-%, particularly 80 to 98 wt.-%, more preferably 90 to 96 wt.-% of filler.

The second tablet layer can generally comprise additional pharmaceutically acceptable excipients. Additional excipients suitable for use in the second tablet layer may include binders, surfactants, lubricants, glidants and coloring agents.

Suitable binders for use in the second tablet layer include povidone (polyvinylpyrrolidone), copovidone (vinylpyrrolidone-vinylacetate copolymer), polyvinyl alcohol, graft copolymer of polyethyleneglycol and polyvinyl alcohol obtainable as Kollicoat IR, polyethylene glycol having a molecular weight in the range of 200 to 10,000, preferably in the range 1,000 to 8,000, cellulose powder, crystalline cellulose, microcrystalline cellulose, siliconized microcrystalline cellulose, cellulose derivatives such as hydroxymethylcellulose, hydroxyethylcellulose, hydroxypropylcellulose and hydroxypropylmethylcellulose, starch, pregelatinized starch, polymethacrylates and mixtures thereof. In particular, the second tablet layer can comprise 0.1 to 20 wt.-% like 0.5 to 20 wt.-% or 1 to 20 wt.-%, particularly 2 to 10 wt.-%, more preferably 3 to 7 wt.-% of binder. Preferably, water soluble binders such as povidone (polyvinylpyrrolidone), copovidone (vinylpyrrolidone-vinylacetate copolymer), polyvinyl alcohol, graft copolymer of polyethyleneglycol and polyvinyl alcohol obtainable as Kollicoat IR, polyethyleneglycol with molecular weight in the range of 200 to 10,000, preferably in the range 1,000 to 8,000, hydroxypropylmethylcellulose having a viscosity (2 wt/vol% solution in water at 25°C) of less than 50 mPas, preferably of less than 15 mPas can be used. More preferably, hydroxypropylcellulose, such as hydroxypropylcellulose having a viscosity of about 100 to 800 mPas when measured in a 10 wt.-% solution in ethanol and determined at 25°C using a Brookfield LVF viscosimeter with spindle, is used as binder in the second tablet layer. It is particularly preferred that the second tablet layer comprises 0.1 to 1 wt.-% of hydroxypropylcellulose.

Suitable surfactants for use in the second tablet layer include anionic, cationic, ampholytic and non-ionic surfactants such as sodium lauryl sulfate, cetrimide, N-dodecyl-N,N-dimethylbetaine, polysorbates (such as Tween®), poloxamers and mixtures thereof. Non-ionic surfactants such as polysorbates and poloxamers are preferred. In particular, the second tablet layer can comprise 1 to 30 wt.-%, particularly 2 to 10 wt.-%, more preferably 3 to 7 wt.-% of surfactant.

Suitable lubricants for use in the second tablet layer include stearic acid or stearic acid salts, such as magnesium stearate, magnesium palmitate and magnesium oleate, hydrogenated vegetable oil, hydrogenated castor oil, talc, sodium stearyl fumarate, macrogols and mixtures thereof. Stearic acid, magnesium stearate and hydrogenated vegetable oil are particularly preferred. In particular, the second tablet layer can comprise 0.1 to 10 wt.-%, particularly 0.25 to 5 wt.-%, more preferably 0.5 to 2 wt.-% of lubricant, such as sodium stearyl fumarate.

Suitable glidants for use in the second tablet layer include colloidal silicon dioxide and magnesium trisilicate. In particular, the second tablet layer can comprise 0.1 to 10 wt.-%, particularly 0.25 to 5 wt.-%, more preferably 0.5 to 2 wt.-% of glidant.

Suitable coloring agents for use in the second tablet layer include dyes and pigments such as iron oxide and titanium oxide. In particular, the second tablet layer can comprise 0.001 to 1 wt.-%, particularly 0.01 to 0.5 wt.-%, more preferably 0.1 to 0.4 or 0.05 to 0.2 wt.-% of coloring agent.

According to a preferred embodiment, the second tablet layer comprises
(aa) 1 to 50 wt.-%, particularly 2 to 20 wt.-%, more preferably 4 to 8 wt.-% like 4 to 9 wt.-% of a diuretic, preferably HCTZ;
(bb) 50 to 99 wt.-%, particularly 80 to 98 wt.-%, more preferably 90 to 96 wt.-% of filler;
(cc) 0 to 30 wt.-%, particularly 2 to 10 wt.-%, more preferably 3 to 7 wt.-% of surfactant;
(dd) 0 to 10 wt.-%, particularly 0.25 to 5 wt.-%, more preferably 0.5 to 2 wt.-% of lubricant;
(ee) 0 to 10 wt.-%, particularly 0.25 to 5 wt.-%, more preferably 0.5 to 2 wt.-% of glidant; and
(ff) 0 to 1 wt.-%, particularly 0.01 to 0.5 wt.-%, more preferably 0.05 to 0.2 wt.-% of coloring agent.

According to another preferred embodiment, the second tablet layer comprises
(aa) 1 to 50 wt.-%, particularly 2 to 20 wt.-%, more preferably 4 to 8 wt.-% like 4 to 9 wt.-% of a diuretic, preferably HCTZ;
(bb) 50 to 99 wt.-%, particularly 80 to 98 wt.-%, more preferably 88 to 94 wt.-% of filler, preferably selected from a sugar alcohol like mannitol;
(cc) 0 to 5 wt.-%, particularly 0.1 to 3 wt.-%, more preferably 0.1 to 1.5 wt.-% of binder, preferably selected from povidone, copovidone, polyvinyl alcohol, graft copolymer of polyethyleneglycol and polyvinyl alcohol obtainable as Kollicoat IR, polyethylene glycol having a molecular weight in the range of 200 to 10,000, preferably in the range 1,000 to 8,000, cellulose powder, crystalline cellulose, microcrystalline cellulose, siliconized microcrystalline cellulose, cellulose derivatives such as hydroxymethylcellulose, hydroxyethylcellulose, hydroxypropylcellulose and hydroxypropylmethylcellulose, polymethacrylates and mixtures thereof, and more preferably hydroxypropylcellulose;
(dd) 0 to 10 wt.-%, particularly 0.25 to 5 wt.-%, more preferably 0.5 to 3 wt.-% of lubricant, preferably selected from stearic acid, stearic acid salts, hydrogenated vegetable oil, hydrogenated castor oil, talc, sodium stearyl fumarate, macrogols and mixtures thereof;
(ee) 0 to 10 wt.-%, particularly 0.01 to 5 wt.-%, more preferably 0.05 to 0.5 wt.-% of glidant, preferably selected from colloidal silicon dioxide and magnesium trisilicate; and
(ff) 0 to 1 wt.-%, particularly 0.01 to 0.8 wt.-%, more preferably 0.1 to 0.7 wt.-% of coloring agent.

Other pharmaceutically acceptable excipients can also be included in the second tablet layer. However, it is generally preferred that the second tablet layer does not comprise substantial amounts of disintegrant. It is further preferred that the second tablet layer comprises less than 2 %, particularly less than 1 %, more preferably less than 0.5 %, most preferably less than 0.1 % by weight of the second tablet layer of a disintegrant. According to a particularly preferred embodiment, the second tablet layer is substantially free of disintegrant.

The term "disintegrant" as used herein refers to any material that has wicking and/or swelling properties when it comes in contact with water. In particular, the term "disintegrant" refers to the group of compounds consisting of crospovidone, pregelatinized starch, sodium starch glycolate, hydroxypropyl starch, carboxymethylcellulose sodium and calcium, crosslinked carboxymethylcellulose sodium, polacrilin potassium, low-substituted hydroxypropylcellulose, sodium and calcium alginate, docusate sodium, methylcellulose, agar, guar gum, chitosan and alginic acid. More particularly, the term "disintegrant" refers to the group of compounds consisting of povidone, crospovidone, starch, pregelatinized starch, sodium starch glycolate, hydroxypropyl starch, microcrystalline cellulose, carboxymethylcellulose sodium and calcium, crosslinked carboxymethylcellulose sodium, polacrilin potassium, low-substituted hydroxypropylcellulose, sodium and calcium alginate, docusate sodium, methylcellulose, agar, guar gum, chitosan and alginic acid.

According to a particular embodiment, the second tablet layer consists of a diuretic and at least one filler.

According to another particular embodiment, the second tablet layer comprises at least one binder.

According to another particular embodiment, the second tablet layer comprises granules and an extragranular phase. Preferably, the granules comprise a diuretic, and in particular hydrochlorothiazide, at least one filler, at least one binder and optionally further excipients like a coloring agent. Moreover, it is preferred that the extragranular phase of the second tablet layer according to this embodiment comprises at least one filler, at least one lubricant and optionally additional excipients such as at least one coloring agent and at least one glidant. More preferably, the extragranular phase comprises at least one filler, at least one lubricant, at least one coloring agent and at least one glidant. The at least one filler of the extragranular phase can be the same as or different from the filler of the granules. Preferably, the filler of the extragranular phase is the same as the filler of the granules. Typically, preferred embodiments with regard to specific components and their amounts are described above. In particular, the filler used in the granules and in the extragranular phase is a sugar alcohol and more particularly mannitol. It is further preferred that the combined weight of the diuretic, the at least one filler of the granules and the at least one filler of the extragranular phase is at least 94 wt.-%, more preferably at least 95 wt.-% and most preferably at least 96 wt.-% such as about 97 wt.-% of the second tablet layer. Accordingly, it is preferred that the combined weight of the diuretic and the at least one sugar alcohol, such as mannitol, of the granules and the extragranular phase is at least 94 wt.-%, more preferably at least 95 wt.-% and most preferably at least 96 wt.-% such as about 97 wt.-% of the second tablet layer. It is also preferred that the combined weight of the filler, and in particular the sugar alcohol such as mannitol, of the granules and the filler, and in particular the sugar alcohol such as mannitol, of the extragranular phase is at least 85 wt.-%, preferably at least 87 wt.-%, more preferably at least 88 wt.-% like at least 89 wt.-% or at least 92 wt.-% of the second tablet layer.

The weight ratio of the filler, and in particular the sugar alcohol such as mannitol, of the granules to the filler, and in particular the sugar alcohol such as mannitol, of the extragranular phase is ranging from 1:1 to 1:10, more preferably from 1:3 to 1:6, and most preferably from 1:4 to 1:5.

Moreover, the weight ratio of the diuretic, and in particular the hydrochlorothiazide, of the granules to the filler, and in particular the sugar alcohol such as mannitol, of the granules is preferably ranging from 1:1 to 1:10, more preferably from 1:1.5 to 1:6, and most preferably from 1:2 to 1:4.5.

Furthermore, the weight ratio of the binder, and in particular hydroxypropylcellulose, of the granules to the filler, and in particular the sugar alcohol such as mannitol, of the granules is preferably ranging from 1:8 to 1:100, more preferably from 1:20 to 1:50, and most preferably from 1:30 to 1:40.

It is also preferred that the weight ratio of the binder, and in particular hydroxypropylcellulose, of the granules to the filler, and in particular the sugar alcohol such as mannitol, of the granules and the extragranular phase, i.e. the weight ratio of the amount of binder to the total amount of filler, is ranging from 1:50 to 1:400, more preferably from 1:130 to 1:280, and most preferably from 1:150 to 1:250.

Accordingly, the second tablet layer preferably comprises
(A) granules comprising, based on the total weight of the second tablet layer,
   (aa) 1 to 50 wt.-%, particularly 2 to 20 wt.-%, more preferably 4 to 8 wt.-% like 4 to 9 wt.-% of a diuretic, preferably HCTZ;
   (bb) 5 to 50 wt.-%, particularly 10 to 30 wt.-%, more preferably 15 to 20 wt.-% of filler, preferably selected from a sugar alcohol like mannitol;
   (cc) 0 to 5 wt.-%, particularly 0.1 to 3 wt.-%, more preferably 0.1 to 1.5 wt.-% of binder, preferably selected from povidone, copovidone, polyvinyl alcohol, graft copolymer of polyethyleneglycol and polyvinyl alcohol obtainable as Kollicoat IR, polyethylene glycol having a molecular weight in the range of 200 to 10,000, preferably in the range 1,000 to 8,000, cellulose powder, crystalline cellulose, microcrystalline cellulose, siliconized microcrystalline cellulose, cellulose derivatives such as hydroxymethylcellulose, hydroxyethylcellulose, hydroxypropylcellulose and hydroxypropylmethylcellulose, polymethacrylates and mixtures thereof, and more preferably hydroxypropylcellulose; and
   (dd) 0 to 1 wt.-%, particularly 0.01 to 0.8 wt.-%, more preferably 0.05 to 0.3 wt.-% of coloring agent, preferably selected from iron oxide and titanium dioxide;
   and
(B) an extragranular phase comprising, based on the total weight of the second tablet layer,
   (aa) 30 to 90 wt.-%, particularly 50 to 85 wt.-%, more preferably 65 to 80 wt.-% of filler, preferably selected from a sugar alcohol like mannitol;
   (bb) 0 to 10 wt.-%, particularly 0.25 to 5 wt.-%, more preferably 0.5 to 3 wt.-% of lubricant, preferably selected from stearic acid, stearic acid salts, hydrogenated vegetable oil, hydrogenated castor oil, talc, sodium stearyl fumarate, macrogols and mixtures thereof;
   (cc) 0 to 10 wt.-%, particularly 0.01 to 5 wt.-%, more preferably 0.05 to 0.5 wt.-% of glidant, preferably selected from colloidal silicon dioxide and magnesium trisilicate; and
   (dd) 0 to 1 wt.-%, particularly 0.01 to 0.8 wt.-%, more preferably 0.1 to 0.7 wt.-% of coloring agent, preferably selected from iron oxide and titanium dioxide.

More preferably, the second tablet layer comprises
(A) granules comprising, based on the total weight of the second tablet layer,
   (aa) 1 to 50 wt.-%, particularly 2 to 20 wt.-%, more preferably 4 to 8 wt.-% like 4 to 9 wt.-% of HCTZ;
   (bb) 5 to 50 wt.-%, particularly 10 to 30 wt.-%, more preferably 15 to 20 wt.-% of filler, preferably a sugar alcohol like mannitol;
   (cc) 0 to 5 wt.-%, particularly 0.1 to 3 wt.-%, more preferably 0.1 to 1.5 wt.-% of binder, preferably hydroxypropylcellulose; and
   (dd) 0 to 1 wt.-%, particularly 0.01 to 0.8 wt.-%, more preferably 0.05 to 0.3 wt.-% of coloring agent, preferably iron oxide;
   and
(B) an extragranular phase comprising, based on the total weight of the second tablet layer,
   (aa) 30 to 90 wt.-%, particularly 50 to 85 wt.-%, more preferably 65 to 80 wt.-% of filler, preferably a sugar alcohol like mannitol;
   (bb) 0 to 10 wt.-%, particularly 0.25 to 5 wt.-%, more preferably 0.5 to 3 wt.-% of lubricant, preferably sodium stearyl fumarate,;
   (cc) 0 to 10 wt.-%, particularly 0.01 to 5 wt.-%, more preferably 0.05 to 0.5 wt.-% of glidant, preferably colloidal silicon dioxide; and
   (dd) 0 to 1 wt.-%, particularly 0.01 to 0.8 wt.-%, more preferably 0.1 to 0.7 wt.-% of coloring agent, preferably iron oxide.

Where the multilayer pharmaceutical tablet according to the invention comprises more than two layers, various embodiments are possible with respect to the composition and order of arrangement of layers. For instance, where the multilayer pharmaceutical tablet is a three-layer tablet, the tablet comprises an additional layer in addition to a first tablet layer and a second tablet layer as defined above. The additional layer can be a further first tablet layer or a further second tablet layer as defined above. For instance, a three-layer tablet can have one first tablet layer arranged between two second tablet layers or one second tablet layer arranged between two first tablet layers. Alternatively, the additional layer can be a layer differing in composition from both the first tablet layer as well as the second tablet layer. For instance, the additional layer can be a telmisartan containing layer of different composition than the first tablet layer or a diuretic containing layer of different composition that the second tablet layer. It can also be a layer containing neither telmisartan nor diuretic. The same applies mutatis mutandis to a multilayer tablet having more than three layers.

According to one embodiment, the multilayer pharmaceutical tablet is a bilayer tablet having a first tablet layer comprising telmisartan and a second tablet layer comprising a diuretic as defined above. According to another embodiment, the multilayer pharmaceutical tablet is a three-layer tablet having a first tablet layer comprising telmisartan arranged between two second tablet layers comprising a diuretic.

The multilayer pharmaceutical tablet according to the invention can be prepared in principle by any method used for manufacturing tablet formulations. Preparation of the tablet generally comprises preparing a first tablet layer composition comprising telmisartan and a second tablet layer composition comprising a diuretic and compressing the tablet layer compositions to produce a multilayer tablet.

The first tablet layer composition comprising telmisartan can be prepared by various methods. Suitable methods include spray-drying and fluid-bed granulation.

One method for preparing the first tablet layer composition comprises preparing a spray-solution by dissolving telmisartan together with at least one basic agent in an appropriate solvent (e.g. water or organic solvent). Optionally, additional excipients, such as a crystallization retarder and/or a surfactant can be included in the spray-solution. The spray solution is subsequently spray-dried to give a spray-dried granulate. The spray-dried granulate is mixed with further excipients to give a tablet layer composition ready for tableting.

Another method for preparing the first tablet layer composition comprises preparing a granulation liquid by dissolving telmisartan together with at least one basic agent in an appropriate solvent (e.g. water or organic solvent). Optionally, additional excipients, such as a crystallization retarder and/or a surfactant can be included in the spray-solution. At least one excipient selected from fillers, binders and mixtures thereof is placed into a fluid-bed granulating machine and sprayed with the granulation liquid. When granulation is completed, the granulate is dried and optionally mixed with further excipients to give a tablet layer composition ready for tableting.

The second tablet layer composition comprising a diuretic can also be prepared by various methods such as direct compression, compression of a granulate obtained by state of the art processes like wet, dry or thermoplastic granulation or melt extrusion. For instance, the second tablet layer composition can in a first embodiment of the present invention be prepared using a wet granulation technique. A suitable wet granulation technique comprises preparing a granulation liquid by dissolving at least a part of a filler and/or binder in an appropriate solvent (e.g. water, organic solvent or a mixture thereof) and adding like spraying the granulation liquid onto a premix comprising diuretic and optionally additional excipients. The obtained wet granulate is dried, optionally sieved and optionally mixed with further excipients to give a tablet layer composition ready for tableting.

Thus, the invention also relates to a process for the preparation of a multilayer pharmaceutical tablet comprising at least one layer comprising telmisartan and at least one layer comprising a diuretic, the process comprising
(a) providing a first tablet layer composition comprising telmisartan;
(b) providing a second tablet layer composition comprising a diuretic by
   (i) granulating the diuretic with at least one filler, at least one binder and optionally other pharmaceutically acceptable excipients to obtain granules; and
   (ii) blending the obtained granules with at least one filler and optionally other pharmaceutically acceptable excipients;
      wherein the weight ratio of the at least one filler of the granules to the at least one filler used in step (b)(ii) is ranging from 1:1 to 1:10;
(c) optionally providing one or more further tablet layer compositions; and
(d) compressing said tablet layer compositions to result in the multilayer pharmaceutical tablet.

Preferably, the granulating in step (b) (i) is a wet granulation step. In particular, the at least one binder is dissolved in a granulation liquid like water and the obtained solution is used to granulate the diuretic, the at least one filler and the optional other excipients such as a coloring agent. The obtained granules are optionally dried and/or sieved prior to the blending in step (b)(ii). The optional other pharmaceutically acceptable excipients used in step (b) (ii) preferably include at least one glidant, at least one lubricant and at least one coloring agent.

Preferred embodiments of the first tablet layer composition comprising telmisartan with regard to specific components and their amounts as well as to methods for its preparation are as described above.

Preferred embodiments of the second tablet layer composition with regard to specific components and their amounts are as described above.

In order to prepare the final multilayer pharmaceutical tablet, the first and second tablet layer compositions and optionally further tablet layer compositions are compressed in a multilayer tableting mode as described in more detail below.

The invention also relates to a multilayer pharmaceutical tablet prepared by the process according to the invention.

According to a second embodiment, the second tablet layer composition is prepared using a dry granulation method as described below.

Thus, the invention also relates to a process for the preparation of a multilayer pharmaceutical tablet comprising at least one layer comprising telmisartan and at least one layer comprising a diuretic, the process comprising
(a) providing a first tablet layer composition comprising telmisartan;
(b) providing a second tablet layer composition comprising a diuretic by
   (i) dry granulating the diuretic with at least one filler and optionally other pharmaceutically acceptable excipients to obtain a granulate; and
   (ii) blending the obtained granulate with at least one filler and optionally other pharmaceutically acceptable excipients;
      wherein the weight ratio of the at least one filler of the granulate to the at least one filler used in step (6)(ii) is ranging from 1:1 to 1:10;
(c) optionally providing one or more further tablet layer compositions; and
(d) compressing said tablet layer compositions to result in the multilayer pharmaceutical tablet.

Preferred embodiments of the first tablet layer composition comprising telmisartan with regard to specific components and their amounts as well as to methods for its preparation are as described above.

Preferred embodiments of the second tablet layer composition with regard to specific components and their amounts are as described above.

It has surprisingly been found that a process for the preparation of a multilayer tablet, wherein a dry granulation method is used for preparing the second tablet layer composition comprising a diuretic, provides a number of advantages. Such process is particularly suitable for application on an industrial scale. In particular, the process is simple and highly cost effective. The tablet layer composition ready for tableting which is obtained according to the process exhibits excellent physical characteristics particularly with regard to flowability and provides for very fast dissolution of the diuretic in the final tablet.

In particular, the second tablet layer composition is prepared by mixing the diuretic and at least one filler in a suitable mixer in order to prepare a pre-mixture. The pre-mixture is then compacted on a dry granulation machine and the compact milled into granules of appropriate particle size. It is preferred that the compact is milled into granules having an average particle size of less than 80 µm, particularly less than 50 µm, more preferably less than 30 µm.

In order to prepare the final multilayer pharmaceutical tablet, the first and second tablet layer compositions and optionally further tablet layer compositions are compressed in a multilayer tableting mode as described in more detail below.

The invention also relates to a multilayer pharmaceutical tablet prepared by the process according to the invention.

According to a third embodiment, the second tablet layer composition is prepared using a thermoplastic granulation, wherein a mixture of diuretic and least one exipient is granulated with a binder having a melting or softening point below 170°C, preferably below 150°C and most preferably below 130°C. Suitable binders having low melting or softening point can be selected from polymers such as, but not limited to, polyethylene glycol having a molecular weight in the range 1,000 to 10,000, poloxamer, povidone with a K value of less than 50, copovidone, Soluplus^{®} or mixtures thereof. State of the art equipment such as high share mixer, fluid bed granulator or extruder can be used in preparation of thermoplastic granulates. Agglomeration can be achieved by adding melted binder or by in situ melting or softening of a binder having low melting or softening point. In situ melting can be achieved by heating the components to the temperature of at least 5 degrees (°C) above the melting or softening temperature of a binder or by heat release due to mixing the powders or elevated pressure. The obtained granulate can optionally be mixed with further excipients and compressed into multilayer tablet.

According to a fourth embodiment, the second tablet layer composition is prepared using a direct compression method where a powder premix of diuretic and at leas one further exicient selected from filler, binder, lubricant, glidant and mixtures thereof is directly compressed onto the first layer containing telmisartan.

In order to prepare the final multilayer pharmaceutical tablet according to the present invention, the first and second tablet layer compositions can be compressed in a manner generally known in the art, such as using a rotary tablet press in an appropriate multilayer tableting mode. For instance, for the manufacture of a bilayer tablet, the first tablet layer composition comprising telmisartan can be introduced into the tableting machine first, followed by introducing the second tablet layer composition comprising diuretic and compressing the two layers in a bilayer tableting mode. Likewise, for the manufacture of a three layer tablet, part of the second tablet layer composition comprising diuretic can be introduced into the tableting machine first, followed by introducing the first tablet layer composition comprising telmisartan and then the remaining second tablet layer composition comprising diuretic and compressing all three layers in a three layer tableting mode.

The invention will be further illustrated by way of the following examples.

### Examples

### Comparative Example 1: Bilayer tablet - Dry granulation

Bilayer tablet composition comprising 80 mg telmisartan and 12.5 mg HCTZ:

| **Telmisartan layer** | [mg] |
|---|---|
| Telmisartan | 80.0 |
| Meglumine | 24.0 |
| Sodium hydroxide | 6.7 |
| Povidone | 24.0 |
| Lactose monohydrate | 120.0 |
| Sorbitol | 299.7 |
| Magnesium stearate | 5.6 |
| **Total telmisartan layer** | **560.0** |
| | |

| **HCTZ layer** | [mg] |
|---|---|
| Hydrochlorothiazide | 12.5 |
| Mannitol | 187.5 |
| **Total HCTZ layer** | **200.0** |

Telmisartan, meglumine, sodium hydroxide and povidone were dissolved in water q.s. in order to prepare a granulation liquid. Lactose monohydrate was placed in a fluid-bed granulating machine and sprayed with the granulation liquid. After granulation had been completed, the granulate was dried and mixed with sorbitol and magnesium stearate to form a tablet layer composition ready for tableting.

Hydrochlorothiazide (HCTZ) was mixed with mannitol and compacted in a dry granulation apparatus. The obtained compact was milled into granules having an average particle size (volume mean diameter) of less than 30 µm. The particle size was determined by laser light scattering using a Malvern Mastersizer Apparatus MS 2000 equipped with a Hydro S dispersion unit. Vegetable oil was used as the dilution medium. Thus, a tablet layer composition containing only HCTZ and mannitol was obtained.

Bilayer tablets containing telmisartan and HCTZ were prepared by first introducing the telmisartan containing tablet layer composition into the tableting machine, followed by the HCTZ containing tablet layer composition. These two layers were then compressed on a rotary tablet press in a bilayer tableting mode.

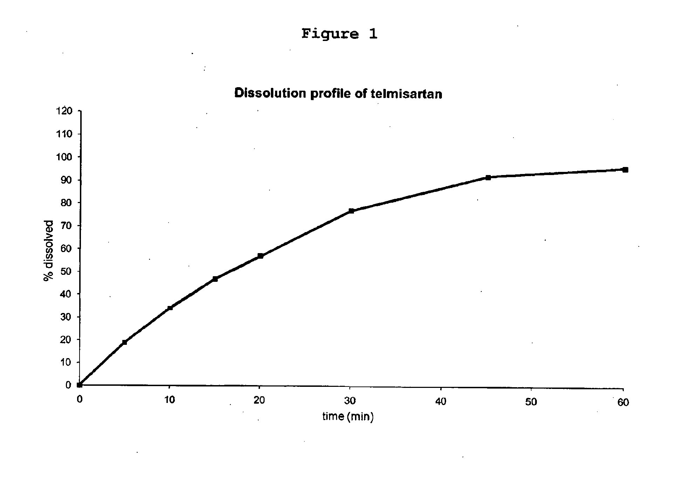

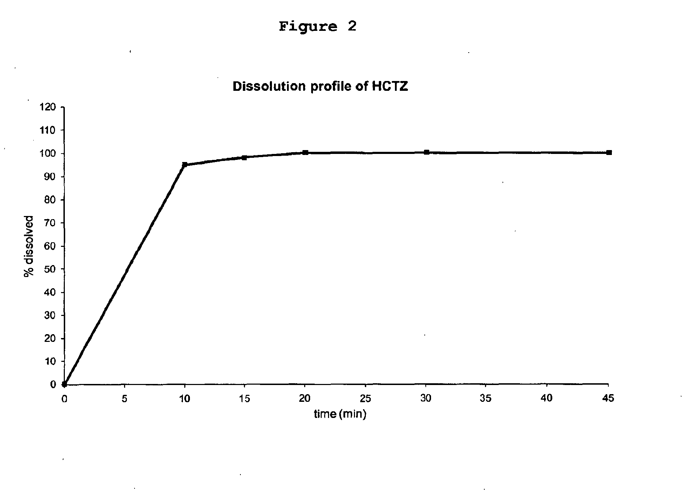

The dissolution profile of the finished bilayer tablet was determined in simulated gastric fluid (pH 2.0, USP apparatus II, 50 rpm). The dissolution profiles thus obtained are shown in Figure 1 (dissolution profile of telmisartan) and Figure 2 (dissolution profile of HCTZ). The dissolution profiles are comparable to that of commercially available Micardis Plus tablets.

### Example 2: Bilayer tablet - Fluid bed granulation

The telmisartan layer was the same as from Example 1.

| **HCTZ layer** | A [mg] | B [mg] | C [mg] | D [mg] |
|---|---|---|---|---|
| Hydrochlorothiazide | 25.00 | 25.00 | 25.00 | 25.00 |
| Mannitol | 25.00 | 119.55 | 119.55 | 25.00 |
| Hydroxypropyl cellulose | 1.00 | 2.25 | | |
| Povidone K30 | | | 2.25 | 1.00 |
| | | | | |
| Mannitol | 95.80 | | | 95.80 |
| Aerosil 200 | 0.20 | 0.20 | 0.20 | 0.20 |
| Magnesium stearate | 3.00 | 3.00 | 3.00 | 3.00 |
| Total HCTZ layer | 150.0 | 150.0 | 150.0 | 150.0 |

Hydrochlorothiazide and mannitol were granulated with hydroxypropyl cellulose or povidone dissolved in water. The obtained granulate was dried in a fluid bed dryer. The obtained granulate was then mixed with additional mannitol (if applicable), Aerosil 200 and magnesium stearate to form final composition ready for tableting.

Bilayer tablets containing telmisartan and HCTZ were prepared by first introducing the telmisartan containing mixture ready for tableting into the tableting machine, followed by introducing the HCTZ containing mixture ready for tableting. These two layers were then compressed on a rotary tablet press in a bilayer tableting mode.

### Comparative Example 3: Bilayer tablet - Direct compression

The telmisartan layer was the same as from Example 1.

| **HCTZ layer** | A [mg] | B [mg] | C [mg] | D [mg] |
|---|---|---|---|---|
| Hydrochlorothiazide | 25.00 | 25.00 | 25.00 | 25.00 |
| Xylitol | 166.60 | 50.00 | | |
| Lactose monohydrate | | | 50.00 | |
| Mannitol | | 116.60 | 116.60 | 166.60 |
| Aeroperl 300 | 0.40 | 0.40 | 0.40 | 0.40 |
| Sodium stearyl fumarate | 8.00 | 8.00 | 8.00 | 8.00 |
| **Total HCTZ layer** | 200.0 | 200.0 | 200.0 | 200.0 |

Hydrochlorothiazide was mixed with either xylitol, lactose monohydrate or mannitol as well as together with Aeroperl 300 and sodium stearyl fumarate to form a final composition ready for tableting by direct compression.

Bilayer tablets containing telmisartan and HCTZ were prepared by firstly introducing the telmisartan containing mixture ready for tableting into the tableting machine, followed by introducing the HCTZ containing mixture ready for tableting. These two layers were then compressed on a rotary tablet press in a bilayer tableting mode.

### Example 4: Bilayer tablet - Wet granulation

| **Telmisartan layer** | A [mg] | B [mg] | C [mg] |
|---|---|---|---|
| Telmisartan | 80.00 | 80.00 | 40.00 |
| Sodium hydroxide | 6.72 | 6.72 | 3.36 |
| Povidone K30 | 24.00 | 24.00 | 12.00 |
| Meglumine | 24.00 | 24.00 | 12.00 |
| Lactose monohydrate, 200 mesh | 120.00 | 120.00 | 60.00 |
| Sorbitol | 299.68 | 299.68 | 149.84 |
| Magnesium stearate | 5.60 | 5.60 | 2.80 |
| Sodium stearyl fumarate | 5.60 | 5. 60 | 2.80 |
| **Total telmisartan layer** | **565.60** | **565.60** | **282.80** |
| | | | |

| **HCTZ layer** | A [mg] | B [mg] | C [mg] |
|---|---|---|---|
| Hydrochlorothiazide | 25.00 | 12.50 | 12.50 |
| Mannitol | 50.00 | 50.00 | 25.00 |
| Iron oxide | 0.40 | 0.40 | 0.40 |
| Hydroxypropyl cellulose | 1.50 | 1.50 | 1.50 |
| | | | |
| Mannitol | 215.85 | 215.85 | 107.93 |
| Iron oxide | 1.05 | 1.05 | 0.52 |
| Colloidal silicon dioxide | 0.20 | 0.20 | 0.10 |
| Sodium stearyl fumarate | 6.00 | 6.00 | 3.00 |
| **Total HCTZ layer** | **300.00** | **287.50** | **150.00** |

Telmisartan, sodium hydroxide and povidone were dissolved in water to obtain a granulation liquid. Lactose monohydrate and meglumine were placed in a fluid-bed granulating machine and sprayed with granulation liquid. When granulation was completed the granulate was dried and mixed with sorbitol, magnesium stearate and sodium stearyl fumarate to form final composition ready for tableting.

HCTZ, mannitol and iron oxide were granulated with a solution of the hydroxypropyl cellulose in water. The obtained granules were dried. The granules were subsequently mixed with the additional mannitol, iron oxide, silicon dioxide and sodium stearyl fumarate to form a final composition ready for tableting.

Bilayer tablets containing telmisartan and HCTZ were prepared by firstly introducing the telmisartan containing mixture ready for tableting into the tabletting machine, which was followed by the HCTZ containing mixture ready for tableting. The two layers were then compressed on a rotary tablet press in a bilayer tableting mode. Telmisartan layer A was combined with HCTZ layer A, telmisartan layer B was combined with HCTZ layer B and telmisartan layer C was combined with HCTZ layer C.

## Claims

1. A multilayer pharmaceutical tablet comprising
(a) at least one first tablet layer comprising
1 to 50 wt.-% telmisartan or a pharmaceutically acceptable salt thereof by weight of the first tablet layer; and
(b) at least one second tablet layer comprising
1 to 50 wt.-% of a diuretic and
50 to 99 wt.-% of at least one filler
by weight of the second tablet layer, wherein the combined weight of the diuretic and the at least one filler is at least 87 wt.-% by weight of the second tablet layer;
wherein the second tablet layer comprises
granules comprising the diuretic, at least one filler, at least one binder and optionally other pharmaceutically acceptable excipients; and
an extragranular phase comprising at least one filler, at least one lubricant and optionally other pharmaceutically excipients;
wherein the weight ratio of the at least one filler of the granules to the at least one filler of the extragranular phase is ranging from 1:1 to 1:10.

2. The tablet according to claim 1, wherein the diuretic of the second tablet layer is selected from the group consisting of amiloride, chlorothalidone, furosemide, hydrochlorothiazide, indapamide and piretanide.

3. The tablet according to claim 1 or 2, wherein the at least one filler of the extragranular phase is the same as the at least one filler of the granules.

4. The tablet according to any one of claims 1 to 3, wherein the at least one filler of the granules and/or the at least one filler of the extragranular phase is a sugar alcohol and preferably is mannitol.

5. The tablet according to any one of claims 1 to 4, wherein the combined weight of the diuretic and the at least one filler of the granules and the at least one filler of the extragranular phase is at least 94 wt.-% of the second tablet layer.

6. The tablet according to any one of claims 1 to 5, wherein the combined weight of the at least one filler of the granules and the at least one filler of the extragranular phase is at least 85 wt.-% of the second tablet layer.

7. The tablet according to any one of claims 1 to 6, wherein the weight ratio of the at least one filler of the granules to the at least one filler of the extragranular phase is ranging from 1:3 to 1:6 and preferably from 1:4 to 1:5.

8. The tablet according to any one of claims 1 to 7, wherein the weight ratio of the diuretic of the granules to the at least one filler of the granules is ranging from 1:1 to 1:10, more preferably from 1:1.5 to 1:6, and most preferably from 1:2 to 1:4.5.

9. The tablet according to any one of claims 1 to 8, wherein the weight ratio of the at least one binder of the granules to the at least one filler of the granules is ranging from 1:8 to 1:100, more preferably from 1:20 to 1:50, and most preferably from 1:30 to 1:40.

10. The tablet according to any one of claims 1 to 9, wherein the weight ratio of the at least one binder of the granules to the at least one filler of the granules and of the extragranular phase is ranging from 1:50 to 1:400, more preferably from 1:130 to 1:280, and most preferably from 1:150 to 1:250.

11. The tablet according to any one of claims 1 to 10, wherein the second tablet layer comprises
(A) granules comprising, based on the total weight of the second tablet layer,
(aa) 1 to 50 wt.-%, particularly 2 to 20 wt.-%, more preferably 4 to 8 wt.-% like 4 to 9 wt.-% of a diuretic, preferably hydrochlorothiazide;
(bb) 5 to 50 wt.-%, particularly 10 to 30 wt.-%, more preferably 15 to 20 wt.-% of filler;
(cc) 0 to 5 wt.-%, particularly 0.1 to 3 wt.-%, more preferably 0.1 to 1.5 wt.-% of binder; and
(dd) 0 to 1 wt.-%, particularly 0.01 to 0.8 wt.-%, more preferably 0.05 to 0.3 wt.-% of coloring agent;
and
(B) an extragranular phase comprising, based on the total weight of the second tablet layer,
(aa) 30 to 90 wt.-%, particularly 50 to 85 wt.-%, more preferably 65 to 80 wt.-% of filler;
(bb) 0 to 10 wt.-%, particularly 0.25 to 5 wt.-%, more preferably 0.5 to 3 wt.-% of lubricant;
(cc) 0 to 10 wt.-%, particularly 0.01 to 5 wt.-%, more preferably 0.05 to 0.5 wt.-% of glidant; and
(dd) 0 to 1 wt.-%, particularly 0.01 to 0.8 wt.-%, more preferably 0.1 to 0.7 wt.-% of coloring agent.

12. The tablet according to claim 11, wherein the second tablet layer comprises
(A) granules comprising, based on the total weight of the second tablet layer,
(aa) 1 to 50 wt.-%, particularly 2 to 20 wt.-%, more preferably 4 to 8 wt.-% like 4 to 9 wt.-% of hydrochlorothiazide;
(bb) 5 to 50 wt.-%, particularly 10 to 30 wt.-%, more preferably 15 to 20 wt.-% of mannitol;
(cc) 0 to 5 wt.-%, particularly 0.1 to 3 wt.-%, more preferably 0.1 to 1.5 wt.-% of hydroxypropylcellulose; and
(dd) 0 to 1 wt.-%, particularly 0.01 to 0.8 wt.-%, more preferably 0.05 to 0.3 wt.-% of iron oxide;
and
(B) an extragranular phase comprising, based on the total weight of the second tablet layer,
(aa) 30 to 90 wt.-%, particularly 50 to 85 wt.-%, more preferably 65 to 80 wt.-% of mannitol;
(bb) 0 to 10 wt.-%, particularly 0.25 to 5 wt.-%, more preferably 0.5 to 3 wt.-% of sodium stearyl fumarate,;
(cc) 0 to 10 wt.-%, particularly 0.01 to 5 wt.-%, more preferably 0.05 to 0.5 wt.-% of colloidal silicon dioxide; and
(dd) 0 to 1 wt.-%, particularly 0.01 to 0.8 wt.-%, more preferably 0.1 to 0.7 wt.-% of iron oxide.

13. The tablet according to any one of claims 1 to 12, which is a three-layer tablet having a first tablet layer comprising telmisartan arranged between two second tablet layers comprising a diuretic.

14. A process for the preparation of a multilayer pharmaceutical tablet comprising at least one layer comprising telmisartan and at least one layer comprising a diuretic, the process comprising
(a) providing a first tablet layer composition comprising telmisartan;
(b) providing a second tablet layer composition comprising a diuretic by
(i) granulating the diuretic with at least one filler, at least one binder and optionally other pharmaceutically acceptable excipients to obtain granules; and
(ii) blending the obtained granules with at least one filler and optionally other pharmaceutically acceptable excipients;
wherein the weight ratio of the at least one filler of the granules to the at least one filler used in step (b)(ii) is ranging from 1:1 to 1:10;
(c) optionally providing one or more further tablet layer compositions; and
(d) compressing said tablet layer compositions to result in the multilayer pharmaceutical tablet.

15. A process for the preparation of a multilayer pharmaceutical tablet comprising at least one layer comprising telmisartan and at least one layer comprising a diuretic, the process comprising
(a) providing a first tablet layer composition comprising telmisartan;
(b) providing a second tablet layer composition comprising a diuretic by
(i) dry granulating the diuretic with at least one filler and optionally other pharmaceutically acceptable excipients to obtain a granulate; and
(ii) blending the obtained granulate with at least one filler and optionally other pharmaceutically acceptable excipients;
wherein the weight ratio of the at least one filler of the granulate to the at least one filler used in step (b)(ii) is ranging from 1:1 to 1:10;
(c) optionally providing one or more further tablet layer compositions; and
(d) compressing said tablet layer compositions to result in the multilayer pharmaceutical tablet.

16. The process according to claim 15, wherein providing the second tablet layer composition in step (b) further comprises (iii) milling the obtained granulate into granules having an average particle size of less than 80 µm, preferably less than 50 µm, more preferably less than 30 µm.

17. A multilayer pharmaceutical tablet prepared by the process according to any one of claims 14 to 16.

## Patentansprüche

1. Pharmazeutische Mehrschichttablette, umfassend:
(a) mindestens eine erste Tablettenschicht, die
1 bis 50 Gew.-%, bezogen auf das Gewicht der ersten Tablettenschicht, Telmisartan oder ein pharmazeutisch annehmbares Salz davon umfasst; und
(b) mindestens eine zweite Tablettenschicht, die
1 bis 50 Gew.-% eines Diuretikums und
50 bis 99 Gew.-%, bezogen auf das Gewicht der zweiten Tablettenschicht, mindestens eines Füllstoffes umfasst, wobei das Gesamtgewicht des Diuretikums und des mindestens einen Füllstoffes mindestens 87 Gew.-%, bezogen auf das Gewicht der zweiten Tablettenschicht, beträgt;
wobei die zweite Tablettenschicht
Granulatkörner, die das Diuretikum, mindestens einen Füllstoff, mindestens ein Bindemittel und gegebenenfalls andere pharmazeutisch annehmbare Hilfsstoffe enthalten; und
eine extragranuläre Phase, die mindestens einen Füllstoff, mindestens ein Gleitmittel und gegebenenfalls weitere pharmazeutische Hilfsstoffe enthält;
umfasst,
wobei das Gewichtsverhältnis des mindestens einen Füllstoffes der Granulatkörner zu dem mindestens einen Füllstoff der extragranulären Phase 1:1 bis 1:10 beträgt.

2. Tablette nach Anspruch 1, wobei das Diuretikum der zweiten Tablettenschicht ausgewählt ist aus der Gruppe bestehend aus Amilorid, Chlortalidon, Furosemid, Hydrochlorthiazid, Indapamid und Piretanid.

3. Tablette nach den Ansprüchen 1 oder 2, wobei der mindestens eine Füllstoff der extragranulären Phase der gleiche ist wie der mindestens eine Füllstoff der Granulatkörner.

4. Tablette nach einem der Ansprüche 1 bis 3, wobei der mindestens eine Füllstoff der Granulatkörner und/oder der mindestens eine Füllstoff der extragranulären Phase ein Zuckeralkohol, bevorzugt Mannit, ist.

5. Tablette nach einem der Ansprüche 1 bis 4, wobei das Gesamtgewicht des Diuretikums und des mindestens einen Füllstoffes der Granulatkörner und des mindestens einen Füllstoffes der extragranulären Phase mindestens 94 Gew.-% der zweiten Tablettenschicht beträgt.

6. Tablette nach einem der Ansprüche 1 bis 5, wobei das Gesamtgewicht des mindestens einen Füllstoffes der Granulatkörner und des mindestens einen Füllstoffes der extragranulären Phase mindestens 85 Gew.-% der zweiten Tablettenschicht beträgt.

7. Tablette nach einem der Ansprüche 1 bis 6, wobei das Gewichtsverhältnis des mindestens einen Füllstoffes der Granulatkörner zu dem mindestens einen Füllstoff der extragranulären Phase 1:3 bis 1:6, bevorzugt 1:4 bis 1:5 beträgt.

8. Tablette nach einem der Ansprüche 1 bis 7, wobei das Gewichtsverhältnis des Diuretikums der Granulatkörner zu dem mindestens einen Füllstoff der Granulatkörner 1:1 bis 1:10, bevorzugter 1:1,5 bis 1:6 und besonders bevorzugt 1:2 bis 1:4,5 beträgt.

9. Tablette nach einem der Ansprüche 1 bis 8, wobei das Gewichtsverhältnis des mindestens einen Bindemittels der Granulatkörner zu dem mindestens einen Füllstoff der Granulatkörner 1:8 bis 1:100, bevorzugter 1:20 bis 1:50 und besonders bevorzugt 1:30 bis 1:40 beträgt.

10. Tablette nach einem der Ansprüche 1 bis 9, wobei das Gewichtsverhältnis des mindestens einen Bindemittels der Granulatkörner zu dem mindestens einen Füllstoff der Granulatkörner und der extragranulären Phase 1:50 bis 1:400, bevorzugter 1:130 bis 1:280 und besonders bevorzugt 1:150 bis 1:250 beträgt.

11. Tablette nach einem der Ansprüche 1 bis 10, wobei die zweite Tablettenschicht
(A) Granulatkörner umfasst, die, bezogen auf das Gesamtgewicht der zweiten Tablettenschicht,
(aa) 1 bis 50 Gew.-%, insbesondere 2 bis 20 Gew.-%, bevorzugter 4 bis 8 Gew.-% wie 4 bis 9 Gew.-% eines Diuretikums, bevorzugt Hydrochlorthiazid;
(bb) 5 bis 50 Gew.-%, insbesondere 10 bis 30 Gew.-%, bevorzugter 15 bis 20 Gew.-% Füllstoff;
(cc) 0 bis 5 Gew.-%, insbesondere 0,1 bis 3 Gew.-%, bevorzugter 0,1 bis 1,5 Gew.-% Bindemittel; und
(dd) 0 bis 1 Gew.-%, insbesondere 0,01 bis 0,8 Gew.-%, bevorzugter 0,05 bis 0,3 Gew.-% Farbstoff
enthalten und
(B) eine extragranuläre Phase umfasst, die, bezogen auf das Gesamtgewicht der zweiten Tablettenschicht,
(aa) 30 bis 90 Gew.-%, insbesondere 50 bis 85 Gew.-%, bevorzugter 65 bis 80 Gew.-% Füllstoff;
(bb) 0 bis 10 Gew.-%, insbesondere 0,25 bis 5 Gew.-%, bevorzugter 0,5 bis 3 Gew.-% Gleitmittel;
(cc) 0 bis 10 Gew.-%, insbesondere 0,01 bis 5 Gew.-%, bevorzugter 0,05 bis 0,5 Gew.-% Fließregulierungsmittel; und
(dd) 0 bis 1 Gew.-%, insbesondere 0,01 bis 0,8 Gew.-%, bevorzugter 0,1 bis 0,7 Gew.-% Farbstoff;
enthält.

12. Tablette nach Anspruch 11, wobei die zweite Tablettenschicht
(A) Granulatkörner umfasst, die, bezogen auf das Gesamtgewicht der zweiten Tablettenschicht,
(aa) 1 bis 50 Gew.-%, insbesondere 2 bis 20 Gew.-%, bevorzugter 4 bis 8 Gew.-% wie 4 bis 9 Gew.-% Hydrochlorthiazid;
(bb) 5 bis 50 Gew.-%, insbesondere 10 bis 30 Gew.-%, bevorzugter 15 bis 20 Gew.-% Mannit;
(cc) 0 bis 5 Gew.-%, insbesondere 0,1 bis 3 Gew.-%, bevorzugter 0,1 bis 1,5 Gew.-% Hydroxypropylcellulose; und
(dd) 0 bis 1 Gew.-%, insbesondere 0,01 bis 0,8 Gew.-%, bevorzugter 0,05 bis 0,3 Gew.-% Eisenoxid
enthalten und
(B) eine extragranuläre Phase umfasst, die, bezogen auf das Gesamtgewicht der zweiten Tablettenschicht,
(aa) 30 bis 90 Gew.-%, insbesondere 50 bis 85 Gew.-%, bevorzugter 65 bis 80 Gew.-% Mannit;
(bb) 0 bis 10 Gew.-%, insbesondere 0,25 bis 5 Gew.-%, bevorzugter 0,5 bis 3 Gew.-% Natriumstearylfumarat;
(cc) 0 bis 10 Gew.-%, insbesondere 0,01 bis 5 Gew.-%, bevorzugter 0,05 bis 0,5 Gew.-% kolloidales Siliciumdioxid; und
(dd) 0 bis 1 Gew.-%, insbesondere 0,01 bis 0,8 Gew.-%, bevorzugter 0,1 bis 0,7 Gew.-% Eisenoxid;
enthält.

13. Tablette nach einem der Ansprüche 1 bis 12, die eine Dreischichttablette ist, die eine erste Tablettenschicht, die Telmisartan enthält, aufweist, die zwischen zwei zweiten Tablettenschichten, die ein Diuretikum aufweisen, angeordnet ist.

14. Verfahren zur Herstellung einer pharmazeutischen Mehrschichttablette, die mindestens eine Schicht, die Telmisartan enthält, und mindestens eine Schicht, die ein Diuretikum enthält, umfasst, bei dem
(a) eine erste Tablettenschichtzusammensetzung, die Telmisartan enthält, zur Verfügung gestellt wird;
(b) eine zweite Tablettenschichtzusammensetzung, die ein Diuretikum enthält, dadurch zur Verfügung gestellt wird, dass
(i) das Diuretikum mit mindestens einem Füllstoff, mindestens einem Bindemittel und gegebenenfalls anderen pharmazeutisch annehmbaren Hilfsstoffen granuliert wird, um Granulatkörner zu erhalten, und
(ii) die erhaltenen Granulatkörner mit mindestens einem Füllstoff und gegebenenfalls anderen pharmazeutisch annehmbaren Hilfsstoffen vermischt werden;
wobei das Gewichtsverhältnis des mindestens einen Füllstoffes der Granulatkörner zu dem mindestens einen Füllstoff, der in Schritt (b)(ii) verwendet wird, 1:1 bis 1:10 beträgt;
(c) gegebenenfalls eine oder mehrere weitere Tablettenschichtzusammensetzungen zur Verfügung gestellt werden; und
(d) die Tablettenschichtzusammensetzungen gepresst werden, um eine pharmazeutische Mehrschichttablette zu ergeben.

15. Verfahren zur Herstellung einer pharmazeutischen Mehrschichttablette, die mindestens eine Schicht, die Telmisartan enthält, und mindestens eine Schicht, die ein Diuretikum enthält, umfasst, bei dem
(a) eine erste Tablettenschichtzusammensetzung, die Telmisartan enthält, zur Verfügung gestellt wird;
(b) eine zweite Tablettenschichtzusammensetzung, die ein Diuretikum enthält, dadurch zur Verfügung gestellt wird, dass
(i) das Diuretikum mit mindestens einem Füllstoff und gegebenenfalls anderen pharmazeutisch annehmbaren Hilfsstoffen trockengranuliert wird, um ein Granulat zu erhalten; und
(ii) das erhaltene Granulat mit mindestens einem Füllstoff und gegebenenfalls anderen pharmazeutisch annehmbaren Hilfsstoffen vermischt wird;
wobei das Gewichtsverhältnis des mindestens einen Füllstoffes des Granulats zu dem mindestens einen Füllstoff, der in Schritt (b) (ii) verwendet wird, 1:1 bis 1:10 beträgt;
(c) gegebenenfalls eine oder mehrere weitere Tablettenschichtzusammensetzungen zur Verfügung gestellt werden; und
(d) die Tablettenschichtzusammensetzungen gepresst werden, um eine pharmazeutische Mehrschichttablette zu ergeben.

16. Verfahren nach Anspruch 15, bei dem das zur Verfügung stellen der zweiten Tablettenschichtzusammensetzung in Schritt (b) zusätzlich (iii) Mahlen des erhaltenen Granulats zu Granulatkörnern mit einer durchschnittlichen Teilchengröße von weniger als 80 µm, bevorzugt weniger als 50 µm, bevorzugter weniger als 30 µm, umfasst.

17. Pharmazeutische Mehrschichttablette, die durch das Verfahren nach einem der Ansprüche 14 bis 16 hergestellt wird.

## Revendications

1. Comprimé pharmaceutique multicouche, comprenant :
a) au moins une première couche de comprimé comprenant de 1 à 50 %, en poids rapporté au poids de la première couche de comprimé, de telmisartan ou d'un sel pharmacologiquement admissible de telmisartan ;
b) et au moins une deuxième couche de comprimé comprenant de 1 à 50 % d'un agent diurétique
et de 50 à 99 % d'au moins une charge,
en poids rapporté au poids de la deuxième couche de comprimé, étant entendu que le poids combiné de l'agent diurétique et de la charge, au nombre d'au moins une, représente au moins 87 % du poids de la deuxième couche de comprimé ;
laquelle deuxième couche de comprimé comprend des granules comprenant l'agent diurétique, au moins une charge, au moins un liant, et en option, d'autres excipients pharmacologiquement admissibles,
et une phase extra-granulaire comprenant au moins une charge, au moins un lubrifiant, et en option, d'autres excipients pharmacologiquement admissibles,
étant entendu que le rapport pondéral de la charge, au nombre d'au moins une, des granules à la charge, au nombre d'au moins une, de la phase extra-granulaire vaut de 1/1 à 1/10.

2. Comprimé conforme à la revendication 1, dans lequel l'agent diurétique de la deuxième couche de comprimé est choisi dans l'ensemble formé par les amiloride, chlorthalidone, furosémide, hydrochlorothiazide, indapamide et pirétanide.

3. Comprimé conforme à la revendication 1 ou 2, dans lequel la charge, au nombre d'au moins une, de la phase extra-granulaire est la même que la charge, au nombre d'au moins une, des granules.

4. Comprimé conforme à l'une des revendications 1 à 3, dans lequel la charge, au nombre d'au moins une, des granules et/ou la charge, au nombre d'au moins une, de la phase extra-granulaire est ou sont un alcool de sucre, et de préférence du mannitol.

5. Comprimé conforme à l'une des revendications 1 à 4, dans lequel le poids combiné de l'agent diurétique, de la charge, au nombre d'au moins une, des granules et de la charge, au nombre d'au moins une, de la phase extra-granulaire représente au moins 94 % du poids de la deuxième couche de comprimé.

6. Comprimé conforme à l'une des revendications 1 à 5, dans lequel le poids combiné de la charge, au nombre d'au moins une, des granules et de la charge, au nombre d'au moins une, de la phase extragranulaire représente au moins 85 % du poids de la deuxième couche de comprimé.

7. Comprimé conforme à l'une des revendications 1 à 6, dans lequel le rapport pondéral de la charge, au nombre d'au moins une, des granules à la charge, au nombre d'au moins une, de la phase extragranulaire vaut de 1/3 à 1/6, et de préférence de 1/4 à 1/5.

8. Comprimé conforme à l'une des revendications 1 à 7, dans lequel le rapport pondéral de l'agent diurétique des granules à la charge, au nombre d'au moins une, des granules vaut de 1/1 à 1/10, mieux encore de 1/1,5 à 1/6, et surtout de 1/2 à 1/4,5.

9. Comprimé conforme à l'une des revendications 1 à 8, dans lequel le rapport pondéral du liant, au nombre d'au moins un, des granules à la charge, au nombre d'au moins une, des granules vaut de 1/8 à 1/100, mieux encore de 1/20 à 1/50, et surtout de 1/30 à 1/40.

10. Comprimé conforme à l'une des revendications 1 à 9, dans lequel le rapport pondéral du liant, au nombre d'au moins un, des granules aux charges, au nombre d'au moins une, des granules et de la phase extra-granulaire vaut de 1/50 à 1/400, mieux encore de 1/130 à 1/280, et surtout de 1/150 à 1/250.

11. Comprimé conforme à l'une des revendications 1 à 10, dans lequel la deuxième couche de comprimé comprend
A) des granules comprenant, par rapport au poids total de la deuxième couche de comprimé,
aa) de 1 à 50 % en poids, en particulier de 2 à 20 % en poids, et mieux encore de 4 à 8 % en poids ou de 4 à 9 % en poids d'un agent diurétique, qui est de préférence de l'hydrochlorothiazide,
bb) de 5 à 50 % en poids, en particulier de 10 à 30 % en poids, et mieux encore de 15 à 20 % en poids d'une charge,
cc) de 0 à 5 % en poids, en particulier de 0,1 à 3 % en poids, et mieux encore de 0,1 à 1,5 % en poids d'un liant,
dd) et de 0 à 1 % en poids, en particulier de 0,01 à 0,8 % en poids, et mieux encore de 0,05 à 0,3 % en poids d'un agent colorant ;
B) et une phase extra-granulaire comprenant, par rapport au poids total de la deuxième couche de comprimé,
aa) de 30 à 90 % en poids, en particulier de 50 à 85 % en poids, et mieux encore de 65 à 80 % en poids d'une charge,
bb) de 0 à 10 % en poids, en particulier de 0,25 à 5 % en poids, et mieux encore de 0,5 à 3 % en poids d'un lubrifiant,
cc) de 0 à 10 % en poids, en particulier de 0,01 à 5 % en poids, et mieux encore de 0,05 à 0,5 % en poids d'un agent de glissement,
dd) et de 0 à 1 % en poids, en particulier de 0,01 à 0,8 % en poids, et mieux encore de 0,1 à 0,7 % en poids d'un agent colorant.

12. Comprimé conforme à la revendication 11, dans lequel la deuxième couche de comprimé comprend
A) des granules comprenant, par rapport au poids total de la deuxième couche de comprimé,
aa) de 1 à 50 % en poids, en particulier de 2 à 20 % en poids, et mieux encore de 4 à 8 % en poids ou de 4 à 9 % en poids d'hydrochlorothiazide,
bb) de 5 à 50 % en poids, en particulier de 10 à 30 % en poids, et mieux encore de 15 à 20 % en poids de mannitol,
cc) de 0 à 5 % en poids, en particulier de 0,1 à 3 % en poids, et mieux encore de 0,1 à 1,5 % en poids d'hydroxypropyl-cellulose,
dd) et de 0 à 1 % en poids, en particulier de 0,01 à 0,8 % en poids, et mieux encore de 0,05 à 0,3 % en poids d'oxyde de fer ;
B) et une phase extra-granulaire comprenant, par rapport au poids total de la deuxième couche de comprimé,
aa) de 30 à 90 % en poids, en particulier de 50 à 85 % en poids, et mieux encore de 65 à 80 % en poids de mannitol,
bb) de 0 à 10 % en poids, en particulier de 0,25 à 5 % en poids, et mieux encore de 0,5 à 3 % en poids de stéaryl-fumarate de sodium,
cc) de 0 à 10 % en poids, en particulier de 0,01 à 5 % en poids, et mieux encore de 0,05 à 0,5 % en poids de dioxyde de silicium colloïdal,
dd) et de 0 à 1 % en poids, en particulier de 0,01 à 0,8 % en poids, et mieux encore de 0,1 à 0,7 % en poids d'oxyde de fer.

13. Comprimé conforme à l'une des revendications 1 à 12, qui est un comprimé à trois couches comportant une première couche de comprimé comprenant du telmisartan, disposée entre deux deuxièmes couches de comprimé comprenant un diurétique.

14. Procédé de fabrication d'un comprimé pharmaceutique multicouche comprenant au moins une couche comprenant du telmisartan et au moins une couche comprenant un agent diurétique, lequel procédé comporte les étapes suivantes :
a) préparer une composition de première couche de comprimé, comprenant du telmisartan ;
b) préparer une composition de deuxième couche de comprimé, comprenant un agent diurétique, en opérant comme suit :
i) granuler l'agent diurétique, avec au moins une charge et au moins un liant, et en option, d'autres excipients pharmacologiquement admissibles, pour en faire des granules,
ii) et mélanger les granules ainsi obtenus avec au moins une charge, et en option, d'autres excipients pharmacologiquement admissibles,
étant entendu que le rapport pondéral de la charge, au nombre d'au moins une, des granules à la charge, au nombre d'au moins une, utilisée dans l'étape (b-ii) vaut de 1/1 à 1/10 ;
c) en option, préparer une ou plusieurs composition(s) supplémentaire(s) de couche de comprimé ;
d) et comprimer lesdites compositions de couche de comprimé, de manière à en faire un comprimé pharmaceutique multicouche.

15. Procédé de fabrication d'un comprimé pharmaceutique multicouche comprenant au moins une couche comprenant du telmisartan et au moins une couche comprenant un agent diurétique, lequel procédé comporte les étapes suivantes :
a) préparer une composition de première couche de comprimé, comprenant du telmisartan ;
b) préparer une composition de deuxième couche de comprimé, comprenant un agent diurétique, en opérant comme suit :
i) granuler à sec l'agent diurétique, avec au moins une charge, et en option, d'autres excipients pharmacologiquement admissibles, pour en faire un granulat,
ii) et mélanger le granulat ainsi obtenu avec au moins une charge, et en option, d'autres excipients pharmacologiquement admissibles,
étant entendu que le rapport pondéral de la charge, au nombre d'au moins une, du granulat à la charge, au nombre d'au moins une, utilisée dans l'étape (b-ii) vaut de 1/1 à 1/10 ;
c) en option, préparer une ou plusieurs composition(s) supplémentaire(s) de couche de comprimé ;
d) et comprimer lesdites compositions de couche de comprimé, de manière à en faire un comprimé pharmaceutique multicouche.

16. Procédé conforme à la revendication 15, dans lequel l'étape (b) de préparation de la composition de deuxième couche de comprimé comprend en outre l'opération suivante :
iii) moudre le granulat obtenu pour en faire des granules présentant une taille moyenne de particules inférieure à 80 µm, de préférence inférieure à 50 µm, et mieux encore inférieure à 30 µm.

17. Comprimé pharmaceutique multicouche, préparé selon un procédé conforme à l'une des revendications 14 à 16.
